# EUROPEAN PATENT SPECIFICATION

(11) **EP 1 206 933 B1**
(45) Date of publication and mention of the grant of the patent: **17.05.2006**
(21) Application number: 01204370.9
(22) Date of filing: 15.11.2001
(51) Int. Cl.: A61K 8/34, A61K 8/42, A61Q 19/00, A61K 47/10, A61K 47/18

(54) **Compositions comprising caprylyl glycol and iodopropynyl butylcarbamate**
Caprylylglykol und Iodopropynylbutylcarbamat enthaltende Zusammensetzungen
Compositions contenant glycol de capryle et iodopropynyl butylcarbamate

(30) Priority: 16.11.2000 EP 00204049
(43) Date of publication of application: 22.05.2002
(73) Proprietor: Johnson & Johnson Consumer France SAS, 92787 Issy les Moulineaux Cedex 9 (FR)
(72) Inventor: David, Gaetane, 78100 Saint Germain en Laye (FR); Magnant, Stéphanie, Hameau de Verdun, 27400 La Vacherie (FR); Porracchia, Jean-François, 78200 Mantes La Jolie (FR)
(74) Representative: Weber-Bruls, Dorothée

(56) References cited:
- EP-A- 1 005 851
- WO-A-98/47469
- PATENT ABSTRACTS OF JAPAN vol. 2000, no. 02, 29 February 2000 (2000-02-29) & JP 11 310506 A (MANDOM CORP), 9 November 1999 (1999-11-09)
- PATENT ABSTRACTS OF JAPAN vol. 1996, no. 06, 28 June 1996 (1996-06-28) & JP 08 053338 A (SHISEIDO CO LTD), 27 February 1996 (1996-02-27)
- PATENT ABSTRACTS OF JAPAN vol. 2000, no. 19, 5 June 2001 (2001-06-05) & JP 2001 048720 A (ASAHI DENKA KOGYO KK), 20 February 2001 (2001-02-20)

## Description

This invention relates to compositions containing caprylyl glycol or an analog thereof, and iodopropynyl butylcarbamate as a preservative agent. It further relates to the use of such compositions in topical formulations, in particular in cosmetic formulations, as well as to the respective topical formulations.

### Background of the invention

Preservatives are agents that are frequently used in cosmetic products, including in personal care products. Preservatives are aimed at protecting products from decay or spoilage, mainly caused by microorganisms. They typically possess anti-microbial activity.

A disadvantage of using such agents is that they may cause adverse effects such as allergic responses and irritation. This is especially the case when applying cosmetic formulations containing preservatives to atopic skins. This is the reason why so far, cosmetic formulations that can be applied to atopic skins do not contain preservatives and therefore are prone to become affected and degraded by microorganisms and moreover to become a source of microbiological contamination.

One would therefore be inclined to reduce the amount of preservative in cosmetic formulations, in particular in those formulations that are or can be used on atopic skins. By doing so, one could expect that the aforementioned side effects are reduced or even are completely absent. However this would result in the concentration of the preservative being too low to be effective so that there is a risk of the formulation becoming affected by microorganisms.

In EP 1 005 851 A1 dermatological and cosmetic compositions are described which comprise at least one alkynyl carbamate and at least one polyol selected from the group consisting of non-etherified polyols, polyalkyl C₁₋₁₀ ethers of polyols, polyalkenyl C₂₋₂₀ ethers of polyols and glycosyl ethers of polyols, wherein these compounds can be saturated, unsaturated, linear, branched, cyclic or can contain a heteroatom or a carbonyl or carboxyl group, 3-(ethyl-2-hexyloxy)-1,2-(propanediol) being most preferred. The composition according to EP 1 005 851 A1 shall be used for the treatment of the scalp, and shall furnish an anti-fungal and an anti-dandruff effect.

In JP 11310506 a cosmetic composition comprising an antiseptic micro-biocide is disclosed which is composed of a combination of at least one kind of paraben and at least one kind selected from 1,2-pentanediol, 1,2 hexanediol and 1,2-octanediol. With the aforementioned diols the anti-microbial potency of parabens shall be increased.

Therefore it would be desirable to make available cosmetic formulations that, on the one hand, have a sufficiently low degree of microbiological contamination and on the other are not susceptible to microbiological contamination and deterioration. It would additionally be desirable to provide such formulations that are devoid of the side effects that are typically caused by preservative agents, in particular in the instance of application to atopic skins.

The compositions and formulations of the present invention meet these goals in that the effect of the preservative agent(s) is potentiated so that less of the preservative needs to be used. Furthermore the potentiating agent has a positive effect on the broadness of the anti-microbial spectrum of the preservative.

Quite unexpectedly it has been found that the use of caprylyl glycol or certain analogs thereof enhance the efficacy of a preservative agent.

Caprylyl glycol in itself is devoid of undesired adversary effects to the skin and in particular to an atopic skin and is currently used as humectant or emollient.

### Summary of the Invention

Thus in one aspect the present invention is concerned with a composition containing caprylyl glycol or an analog thereof, as defined in claim 1, and iodopropynyl butylcarbamate as a preservative agent.

A particularly preferred composition comprises caprylyl glycol (also referred to as 1,2-octanediol) and iodopropynyl butylcarbamate.

In another aspect the invention provides a topical formulation comprising a composition as defined herein and further ingredients. The topical formulation can be for dermatological use, but in particular is for cosmetic use.

In a further aspect, the invention relates to the use of caprylyl glycol or an analog thereof, as defined in claim 1, for potentiating or boosting the effect of iodopropynyl butylcarbamate as a preservative agent. Said use preferably is in topical formulations, more preferably in dermatological or in cosmetic formulations.

The invention further relates to the use of a composition as defined herein as a preservative system. Said use preferably is in topical formulations, more preferably in dermatological or in cosmetic formulations.

In a particular aspect the invention concerns the use as a preservative system of a composition comprising caprylyl glycol or an analog thereof and iodopropynyl butylcarbamate.

In another aspect there is provided a composition as defined herein wherein iodopropynyl butylcarbamate, is present in an amount that is less than effective. Similarly, there is provided a topical formulation as defined herein wherein iodopropynyl butylcarbamate is present in an amount that is less than effective.

### Detailed Description of the Invention

As used herein, caprylyl glycol refers to 1,2-octanediol and can be structurally represented by the formula:

CH₃-(CH₂)₄-CH₂-CH(OH)-CH₂OH

Caprylyl glycol analogs comprise C₆₋₁₂ alkanediols. Preferred are the alkanediols mentioned herein wherein the hydroxy groups are vicinally substituted. Examples of such alkanediols are 2,3-octanediol, 1,2-nonanediol, 1,2-decanediol, 1,2-dodecanediol, 1,2-heptanediol, 1,2-hexanediol, 3,4-octanediol and the like. Of particular interest are those vicinal alkanediols wherein one hydroxy is substituted at an end carbon and the other on the carbon atom next thereto.

A particular group of caprylyl glycol analogs are those which have a 1,2-octanediol skeleton which is further substituted with 1, 2 or 3 C₁₋₄ alkyl groups (or 1,2-octanediol substituted with 1, 2 or 3 C₁₋₄ alkyl groups) such as, for example, 3-methyl-1,2-octanediol, 4-methyl-1,2-octanediol, 3,4-dimethyl octanediol, 3-ethyl-1,2-octanediol, 4-ethyl-1,2-octanediol and the like.

The caprylyl glycol analogs that can be used in the compositions or formulations of the invention preferably are devoid of any adverse effects on the skin, in particular on atopical skins, such adverse effects particularly comprising allergic reactions and irritation.

The term 'caprylyl glycol or an analog thereof' is also meant to comprise mixtures of caprylyl glycol and one or more of its analogs, or mixtures of two or more caprylyl glycol analogs as mentioned herein.

The preservatives that is used in the compositions or topical formulations of the invention is the preservative iodopropynyl butylcarbamate, also referred to as butyl-3-iodo-2-propynylcarbamate or IPBC, that can be structurally represented by the following chemical structure:

I-C≡C-CH₂-O-(C=O)-NH-(CH₂)₃-CH₃

As used herein the term 'preservative' is meant to also comprise mixtures of iodopropynyl butylcarbamate with one or more other preservatives, in particular the preservatives mentioned in the following which comprise phenoxyethanol, alkylparabens and their salts, in particular their alkalimetal salts such as sodium salts (e.g. C₁₋₆ alkyl parabens such as methyl, ethyl, propyl, butyl paraben and the like parabens), chlorexidine, and the like, and mixtures of preservatives such as, for example, Nipaguard^{™} (e.g. Nipaguard MPA^{™}), Phenonipe^{™}, and the like, formaldehyde or formaldehyde releaser, benzyl alcohol, chloroxylenol, methylchloroisothiazolinone, methylisothiazolinone, sodium benzoate, chlorhexidine, digluconate, methyldibromo glutaronitrile, 5-bromo-5-nitro-1,3-dioxane, benzoic acid, dehydroacetic acid, diazolidinyl urea, dichlorobenzyl alcohol, glucose oxidase, hexamidine diisethionate, imidazolidinyl urea, lactoperoxidase, PEG-4 laurate, phenethyl alcohol, polyaminopropyl biguanide, potassium sorbate, propylene glycol, pyridoxine HCl, quaternium-15, sorbic acid, triclosan and the like.

The weight by weight ratio of caprylyl glycol or its analog to iodopropynyl butylcarbamate typically is in the range of 0.1 to 500, in particular 1 to 500, or 1 to 250, or 2 to 500. Preferably said ratio is in the range of 5 to 100, or 10 to 100, or 5 to 75. More preferably said ratio is in the range of 20 to 100, or 20 to 75. Still more preferably said ratio is in the range of 25 to 75 or 50 to 60. In particular said ratio is 55 or about 55.

The amount of caprylyl glycol or its analog in the formulations according to this invention may vary, but will be selected such that the combination thereof with the preservative has an effective preservative activity. It has been found that good results are obtained when using concentrations in the range from about 0.1 to 30% weight/weight (w/w) of the composition. In particular, the concentration of caprylyl glycol or its analog should be in the range from 0.1 to 20 %, more in particular from 0.2 to 10 %, preferably from 0.5 to 5 % or 0.5 to 2 %, all percentages being w/w. A particularly preferred concentration is 1 % w/w.

The term effective preservative activity means that its activity is such that the composition or formulation is protected for a sustained period of time, in particular during the so-called 'shelf life' of the product. The 'shelf-life' of a product is determined according to methods generally known in the art.

The amount of of IPBC in the formulations according to this invention may vary but it has been found that good results are obtained when using concentrations in the range from about 0.001 to 1% w/w of the composition, in particular from 0.001 to 0.5 %, or from 0.001 to 0.25 %, in particular from 0.001 to 0.100 %, or from 0.001 to 0.050 %, all percentages being w/w. A particular preferred concentration is in the range of 0.010 to 0.025 % w/w. Or expressed differently, the concentration of the preservative agent and in particular of IPBC in the formulation is in the range 1 to 250 ppm, or 1 to 200 ppm, more preferably in the range of 10 to 200 ppm, 'ppm' also referring to w/w.

Of special interest is a formulation containing about 1 % w/w of caprylyl glycol and about 0.018 % w/w of IPBC to obtain a broad anti-microbial spectrum that shows better results than IPBC alone. The compositions of the present invention contain caprylyl alcohol or its analog and iodopropynyl butylcarbamate, and optionally other components. Other components may be solvents or any of the other components mentioned hereinafter as components that can be added to the topical formulations according to the invention.

The compositions of the present invention are generally prepared by mixing the caprylyl glycol or the analog thereof, and iodopropynyl butylcarbamate. Solvent may be added after mixing, or the components are mixed while being present in a solvent. Other components may be added during the mixing or afterwards. The said glycol and preservative may also be added to a premix of other components.

This invention further relates to topical formulations containing a composition as defined herein. Topical compositions comprise as well dermatological formulations (or topical pharmaceutical formulations), as cosmetical formulations. Said topical formulations may further contain other ingredients or additives used in dermatological or in cosmetical formulations, including other active ingredients.

The formulations according to the present invention are formulated into forms that are useful in personal care products, especially in emulsions. The formulations are in particular for application to atopic skins.

The topical formulations according to the present invention may additionally contain further ingredients or additives such as solvents, surfactants, emulsifiers, consistency factors, conditioners, emollients, skin caring ingredients, moisturizers, thickeners, lubricants, fillers, anti-oxidants, preservatives, active ingredients, in particular dermatologically active ingredients, fragrances and the like. Active ingredients as mentioned herein comprise, for example, anti-inflammatories, anti-bacterials, anti-fungals and the like agents. Active ingredients suited for topical applications are particularly preferred.

Suitable surfactants comprise:
alkyl sulfates e.g. sodium lauryl sulfate, ammonium lauryl sulfate, sodium cetearyl sulfate,
alkyl sulfoacetates e.g. sodium lauryl sulfoacetate,
alkyl ether sulfates e.g. sodium laureth sulfate, sodium trideceth sulfate, sodium oleth sulfate, ammonium laureth sulfate
alkyl ether sulfosuccinates e.g. disodium laureth sulfosuccinate
alkyl glycosides e.g. decyl glucoside, lauryl glucoside,
alkyl isethionates
amphoterics e.g. cocamidopropyl betaine, sodium cocoamphoacetate, sodium lauroamphoacetate, disodium lauroamphodiacetate, disodium cocoamphodiacetate, sodium lauroamphopropionate, disodium lauroamphodipropionate, potassium or ammonium slats of the aforementioned amphoterics, capryl/capramidopropyl betaine, undecylenamidopropyl betaine, lauramidopropyl betaine and
fatty alcohol polyglycol ethers.

Suitable emulsifiers are e.g. anionics as salts of fatty acids e.g. sodium stearate or sodium palmitate, organic soaps e.g. mono-, di- or triethanolaminoleate, sulfated or sulfonated compounds e.g. sodium lauryl sulfate or sodium cetyl sulfonate, saponines, lamepones; cationics as quaternary ammonium salts; nonionics as fatty alcohols, fatty acid ester with saturated or unsaturated fatty acids, polyoxyethylenesters or polyoxyethylenethers of fatty acids, polymers from ethylene oxide and propylene oxide or propylene glycol, amphotherics as phosphatides, proteines as gelatine, casein alkylamidobetaines, alkyl betaines and amphoglycinates, alkyl phosphates, alkylpolyoxyethylene phoaphates or the corresponding acids, silicone derivatives, e.g. alkyl dimethiconecoplyol.

Suitable consistency factors are e.g. fatty alcohols or their mixtures with fatty acid esters, e.g. acetylated lanolin alcohol, aluminum stearates, carbomer, cetyl alcohol, glyceryl oleate, glyceryl stearate, glyceryl stearate (and) PEG 100 stearate, magnesium stearate, magnesium sulfate, oleic acid, stearic acid, stearyl alcohol, myristyl myristate, isopropyl palmitate, beeswax and synthetic equivalents thereof, carbomers, etc. Suitable conditioners are e.g. alkylamido ammonium lactate, cetrimonium chloride and distearoylethyl hydroxyethylmonium methosulfate and cetearyl alcohol, cetyl dimethicone, cetyl ricinoleate, dimethicone, laureth-23, laureth-4, polydecene, retinyl palmitate, quaternised protein hydrolysates, quaternised cellulose and starch derivatives, quaternised copolymers of acrylic or methacrylic acid or salts, quaternised silicone derivatives.

Suitable emollients are e.g. cetearyl isononanoate, cetearyl octanoate, decyl oleate, isooctyl stearate, coco caprylate/caprate, ethylhexyl hydroxystearate, ethylhexyl isononanoate, isopropyl isostearate, isopropyl myristate, oleyl oleate, hexyl laurate, paraffinum liquidum, PEG-75 lanolin, PEG-7 glyceryl cocoate, petrolatum, ozokerite, cyclomethicone, dimethicone, dimethicone copolyol, dicaprylyl ether, butyrospermum parkii, buxus chinensis, canola, carnauba cera, copernicia cerifera, oenothera biennis, elaeis guineensis, prunus dulcis, squalane, zea mays, glycine soja, helianthus annuus, lanolin, hydrogenated castor oil, hydrogenated coconut oil, hydrogenated polyisobutene, sucrose cocoate, stearoxy dimethicone, lanolin alcohol, isohexadecane.

Suitable skin caring ingredients are e.g. plant extracts, bisabolol, antiinflammatory agents, urea, allantoin, panthenol and panthenol derivatives, phytantriol, vitamines A, E, C, D, ceramides of animal or plant origin, lecithins, etc.

Suitable moisturizers are e.g. butylene glycol, cetyl alcohol, dimethicone, dimyristyl tartrate, glucose, glycereth-26, glycerin, glyceryl stearate, hydrolyzed milk protein, lactic acid, lactose and other sugars, laureth-8, lecithin, octoxyglycerin, PEG-12, PEG-135, PEG-150, PEG-20, PEG-8, pentylene glycol, hexylene glycol, phytantriol, polyquaternium-39, PPG-20 methyl glucose ether, propylene glycol, sodium hyaluronate, sodium lactate, sodium PCA, sorbitol, succinoglycan, synthetic beeswax, tri-C14-15 alkyl citrate, starch.

Suitable thickeners are e.g. acrylates/steareth-20 methacrylate copolymer, carbomer, carboxymethyl starch, cera alba, dimethicone/vinyl dimethicone crosspolymer, propylene glycol alginate, hydroxyethylcellulose, hydroxypropyl methylcellulose, silica, silica dimethyl silylate, xanthan gum, hydrogenated butylene/ethylene/styrene copolymer.

Suitable lubricants are e.g. adipic acid, fumaric acid and its salts, benzoic acid and its salts, glycerine triacetate, sodium or magnesium lauryl sulfate, magnesium stearate, solid polyethylenglycol, polyvinylpyrrolidone, boric acid, monolaurate or - palmitate, myristyl alcohol, cetyl alcohol, cetylstearyl alcohol, talcum, calcium or magnesium salts of higher fatty acids, mono-, di- or triglycerides of higher fatty acids, polytetrafluorethylen.

Suitable anti-oxidants are e.g. sulfites, e.g. sodium sulfite, tocopherol or derivates thereof, ascorbic acid or derivates thereof, citric acid, propyl gallate, chitosan glycolate, cysteine, N-acetyl cysteine plus zinc sulfate, thiosulfates, e.g. sodium thiosulfate, polyphenoles and the like.

The compositions may further contain active ingredients, e.g. anti-microbials such as complexes of PVP and hydrogen peroxide, anti-inflammatories, plant extracts, bisabolol, panthenol, tocopherol, actives for anti-stinging, anti-irritants, anti-dandruffs, or anti-aging agents such as retinol, melibiose and the like. Other suitable actives are e.g. Medicago officinalis, Actinidia chinensis, allantoin, Aloe barbadensis, Anona cherimolia, Anthemis nobilis, Arachis hypogaea, Arnica montana, Avena sativa, beta-carotene, bisabolol, Borago officinalis, butylene glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea arabica, Crataegus monogina, Cucumis melo, dichlorophenyl imidazoldioxolan, Enteromorpha compressa, Equisetum arvense, ethoxydiglycol, ethyl panthenol, farnesol, ferulic acid, Fragaria chiloensis, Gentiana lutea, Ginkgo biloba, glycerin, glyceryl laurate, Glycyrrhiza glabra, Hamamelis virginiana, heliotropine, hydrogenated palm glycerides, citrates, hydrolyzed castor oil, hydrolyzed wheat protein, Hypericum perforatum, Iris florentina, Juniperus communis, Lactis proteinum, lactose, Lawsonia inermis, linalool, Linum usitatissimum, lysine, magnesium aspartate, Magnifera indica, Malva sylvestris, mannitol, mel, Melaleuca alternifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, Prunus armeniaca, Prunus persica, retinyl palmitate, Ricinus communis, Rosa canina, Rosmarinus officinalis, Rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talc, Thymus vulgaris, Tilia cordata, tocopherol, tocopheryl acetate, trideceth-9, triticum vulgare, tyrosine, undecylenoyl glycine, urea, Vaccinium myrtillus, valine, zinc oxide, zinc sulfate.

The combination of caprylyl glycol or an analog thereof and a preservative can be used in emulsions, both oil-in-water, and water-in-oil, in aqueous solutions, in PIT (phase inversion temperature) emulsions, in oily solutions, in foaming cosmetic formulations (foams), and in so-called multiple emulsions, e.g. in triple emulsions (such as water/oil/water emulsions).

The compositions of the invention can be formulated as creams, gels, liquids or lotions. They can be used in shampoos, hair conditioners, hair dyes, hair preparations, aftershave lotions, bath soaps and detergents, fragrance preparations, suncare products, indoor tanning products, body and hand preparations, personal cleansers, shaving preparations, tonics, dressings and other hair grooming aids, moisturizing preparations, skin care preparations and the like.

The topical formulations of the invention are prepared by adding other ingredients to a composition as defined herein, or adding to a mixture of ingredients a composition as defined herein. Alternatively, said formulations may also be made by mixing the ingredients individually or by groupwise mixing. Subsequently other specific ingredients, such as perfumes, may be added.

As used herein the term 'atopic skins' refers to the skin of persons suffering from atopy, or skins prone to atopic disease, or skins showing symptoms of atopic disease.

The formulations of the present invention therefore are useful for application with persons suffering from atopic disease or having an atopic skin. Actually, as atopic skins are very sensitive, the use of caprylyl glycol allows using less preservative molecules known for their allergic or irritating effects.

In a further aspect, this invention is concerned with synergistic effects between two agents, caprylyl glycol or an analog on the one hand, IPBC as the preservative on the other (in particular between caprylyl glycol and IPBC) in terms of anti-microbiological activity, as well as anti-microbiological spectrum, that shown a better efficacy than the two components alone. Hence in still a further aspect the present invention provides synergistic cosmetic compositions comprising caprylyl glycol or an analog and IPBC as a preservative and in particular caprylyl glycol and IPBC. In these synergistic compositions the concentration of caprylyl glycol or an analog, and of IPBC, and the by weight ratio of said glycol or IPBC can be as mentioned herein.

The use of caprylyl glycol or an analog thereof in combination with iodopropynyl butylcarbamate, in particular of 1,2-octanediol with IPBC, in cosmetic formulations results in both a broad anti-microbial protection and a good skin tolerance. The anti-microbial protection is as well against bacteria as fungi, in particular against species such as, for example, Pseudomonas aeruginosa, Escherichia coli, Staphyococcus aureus, Candida albicans, Aspergillus niger and the like and especially against Staphylococcus aureus.

The combinations of the invention are particularly attractive for personal care products, and more specifically those personal care products aimed for application to atopic skins.

The compositions or formulations of the invention are particularly attractive since the agents therein have a good anti-microbial action on Staphylococcus aureus, and because atopic skins are prone to develop this kind of bacteria.

Thus in a further aspect, this invention concerns the use of the formulations as defined herein for combating microorganisms or preventing the growth thereof on human skin, in particular on atopic skin.

Additionally, the combination of the two agents mentioned herein may have pharmaceutical applications and to that purpose may be formulated in appropriate formulations for topical pharmaceutical applications. Hence in still a further aspect the invention provides topical pharmaceutical compositions comprising a composition as defined herein. The composition is present in an amount to effectively preserve the said pharmaceutical compositions.

The invention is further illustrated by the following examples.

### Example 1:

| **Component** | **% w/w** |
|---|---|
| Aqua | 44.900 |
| Carbomer | 0.4000 |
| Hydroxyethylcellulose | 0.2000 |
| | |
| Tromethamine | 0.2500 |
| | |
| Glycerin | 3.8000 |
| PEG 8 | 5.0000 |
| Disodium EDTA | 0.1000 |
| Allantoin | 0.5000 |
| Panthenol 75% / aqua 25% | 0.5000 |
| Grapefruit seed extract 20% / Glycerin 40% / aqua 40% | 3.0000 |
| Butylene glycol | 2.0000 |
| Iodopropynyl butylcarbamate / PEG-4 laurate | 0.0200 |
| Caprylyl glycol | 1.0000 |
| | |
| Potassium cetyl phosphate | 2.0000 |
| PEG 100 stearate 50% / glyceryl steareate 50% | 0.5000 |
| Cetearyl alcohol | 1.4000 |
| Myreth - 3 myristate | 2.8000 |
| Vegetal oil & hydrogenated vegetable oil & candelilla wax | 1.8000 |
| Vegetable oil | 11.500 |
| Dimethicone | 1.4000 |
| Hydrogenated polyisobutene | 8.4000 |
| | |
| Borago officinalis | 4.0000 |
| Canola | 3.7000 |
| Bisabolol | 0.0500 |
| | |
| Tocopherol acetate | 0.1000 |
| Laureth-7 5,5 % / polyacrylamide 40 % / C13-15 isoparafin 20 % / aqua 34,5 % | 0.5000 |

The components of a group in the above list (groups being separated by a space in the above list) are mixed together. Subsequently the group mixtures are added to one another and mixed into the end product. The same procedure is followed for the formulation listed hereafter.

### Example 2:

| | |
|---|---|
| Aqua | 44.900 |
| Carbomer | 0.4000 |
| Hydroxyethylcellulose | 0.2000 |
| | |
| Tromethamine | 0.2500 |
| | |
| Glycerin | 3.8000 |
| PEG 8 | 05.000 |
| Disodium EDTA | 0.1000 |
| Allantoin | 0.5000 |
| Panthenol 75 % / aqua 25 % | 0.5000 |
| Grapefruit seed extract 20 % / Glycerin 40 % / aqua 40 % | 3.0000 |
| Butylene glycol | 2.0000 |
| Iodopropynyl butylcarbamate / PEG-4 laurate | 0.0200 |
| Caprilyl glycol | 1.0000 |
| | |
| Potassium cetyl phosphate | 2.0000 |
| PEG 100 stearate 50% / glyceryl stearate 50% | 0.5000 |
| Cetearyl alcohol | 1.4000 |
| Myreth -3 Myristate | 2.8000 |
| Vegetal oil & hydrogenated vegetable oil & candelilla wax | 1.8000 |
| Vegetable oil | 11.500 |
| Dimethicone | 1.4000 |
| Hydrogenated polyisobutene | 8.4000 |
| | |
| Borago offcinalis | 4.0000 |
| Canola | 3.7000 |
| Bisabolol | 0.0500 |
| | |
| Tocophenol acetate | 0.1000 |
| Laureth-7 5,5 % / polycrylamide 40 % / C13-15 isoparafin 20 % / Aqua 34,5 % | 0.5000 |

## Claims

1. A composition containing caprylyl glycol, C₆₋₁₂ alkanediols or 1,2-octanediol substituted with 1, 2 or 3 C₁₋₄ alkyl groups, wherein the hydroxyl groups are vicinally substituted and wherein one hydroxyl group is substituted at an end carbon and the other on the carbon atom next thereto and mixtures thereof, and iodopropynyl butylcarbamate.

2. A composition according to claim 1 wherein
the C₆₋₁₂ alkanediols comprise 1,2-nonanediol and 1,2-heptanediol.

3. A composition according to claim 1 wherein
the 1,2-octanediol skeleton is further substituted with 1, 2 or 3 C₁₋₄ alkyl groups comprises 3-methyl-1,2-octanediol, 4-methyl-1,2-octanediol, 3,4-dimethyl octanediol, 3-ethyl-1,2,-octanediol and 4-ethyl-1,2-octanediol.

4. A composition according to one of the preceding claims wherein
the ratio w/w of caprylyl glycol, C₆₋₁₂ alkanediol or 1,2-octanediol substituted with 1, 2 or 3 C₁₋₄ alkyl groups and iodopropynyl butylcarbamate is in the range of 0.1 to 500.

5. A composition according to one of the preceding claims wherein
the ratio w/w of caprylyl glycol, C₆₋₁₂ alkanediol or 1,2-octanediol substituted with 1, 2 or 3 C₁₋₄ alkyl groups and iodopropynyl butylcarbamate is in the range of 20 to 75.

6. A composition according to one of the preceding claims wherein
the concentration of caprylyl glycol, C₆₋₁₂ alkanediol or 1,2-octanediol substituted with 1, 2 or 3 C₁₋₄ alkyl groups is in the range from 0.2 to 10 wt.-%.

7. A composition according to one of the preceding claims wherein
the concentration of iodopropynyl butylcarbamate is in the range from 0.01 to 0.25 wt.-%.

8. A composition according to one of the preceding claims containing
about 1 wt.-% of caprylyl glycol and about 0.018 wt.-% of iodopropynyl butylcarbamate.

9. A composition according to one of the preceding claims further comprising surfactants, emulsifiers, consistency factors, conditioners, emollients, skin caring ingredients, moisturizers, thickeners, lubricants, fillers, anti-oxidants, preservatives, fragrances, anti-inflammatories, anti-bacterials or anti-fugals.

10. A topical formulation comprising a composition as claimed in one of the preceding claims.

11. The use of a composition according to claims 1 to 9 in dermatological or in cosmetic formulations.

## Patentansprüche

1. Zusammensetzung, enthaltend Caprylylglykol, C₆₋₁₂-Alkandiole oder 1,2-Octandiol, substituiert mit 1, 2 oder 3 C₁₋₄-Alkylgruppen, wobei die Hydroxylgruppen vicinal substituiert sind und wobei eine Hydroxylgruppe an einem terminalen Kohlenstoff substituiert ist und die andere an dem daneben befindlichen Kohlenstoffatom, und Gemische daraus, sowie Iodopropynylbutylcarbamat.

2. Zusammensetzung nach Anspruch 1, **dadurch gekennzeichnet, dass**
die C₆₋₁₂-Alkandiole 1,2-Nonandiol und 1,2-Heptandiol umfassen.

3. Zusammensetzung nach Anspruch 1, **dadurch gekennzeichnet, dass**
das 1,2-Octandiol-Gerüst, weiterhin substituiert mit 1, 2 oder 3 C₁₋₄-Alkylgruppen, 3-Methyl-1,2-octandiol, 4-Methyl-1,2-octandiol, 3,4-Dimethyloctandiol, 3-Ethyl-1,2-octandiol und 4-Ethyl-1,2-octandiol umfasst.

4. Zusammensetzung nach einem der vorangehenden Ansprüche, **dadurch gekennzeichnet, dass**
das w/w-Verhältnis von Caprylylglycol, C₆₋₁₂-Alkandiol oder 1,2-Octandiol, substituiert mit 1, 2 oder 3 C₁₋₄-Alkylgruppen, zu Iodopropynylbutylcarbamat im Bereich von 0,1 bis 500 liegt.

5. Zusammensetzung nach einem der vorangehenden Ansprüche, **dadurch gekennzeichnet, dass**
das w/w-Verhältnis von Caprylylglycol, C₆₋₁₂-Alkandiol oder 1,2-Octandiol, substituiert mit 1, 2 oder 3 C₁₋₄-Alkylgruppen, zu Iodopropynylbutylcarbamat im Bereich von 20 bis 75 liegt.

6. Zusammensetzung nach einem der vorangehenden Ansprüche, **dadurch gekennzeichnet, dass**
die Konzentration von Caprylylglycol, C₆₋₁₂-Alkandiol oder 1,2-Octandiol, substituiert mit 1, 2 oder 3 C₁₋₄-Alkylgruppen, im Bereich von 0,2 bis 10 Gew.-% liegt.

7. Zusammensetzung nach einem der vorangehenden Ansprüche, **dadurch gekennzeichnet, dass**
die Konzentration von Iodopropynylbutylcarbamat im Bereich von 0,01 bis 0,25 Gew.-% liegt.

8. Zusammensetzung nach einem der vorangehenden Ansprüche, enthaltend etwa 1 Gew.-% Caprylylglycol und etwa 0,018 Gew.-% Iodopropynylbutylcarbamat.

9. Zusammensetzung nach einem der vorangehenden Ansprüche, weiterhin umfassend grenzflächenaktive Stoffe, Emulgatoren, Konsistenzfaktoren, Konditioniermittel, Weichmacher, Hautpflegestoffe, Feuchthaltestoffe, Eindicker, Schmierstoffe, Füllstoffe, Antioxidationsmittel, Konservierungsstoffe, Duftstoffe, entzündungshemmende, antibakterielle oder antifugale Stoffe.

10. Topische Zubereitung, umfassend eine Zusammensetzung nach einem der vorangehenden Ansprüche.

11. Die Verwendung einer Zusammensetzung nach den Ansprüchen 1 bis 9 in dermatologischen oder in kosmetischen Zubereitungen.

## Revendications

1. Composition contenant du caprylyl glycol, des alcanediols en C₆₋₁₂ ou du 1,2-octanediol substitué par 1, 2 ou 3 groupes alkyle en C₁₋₄, dans lequel les groupes hydroxyle sont substitués de façon contiguë et dans lequel un groupe hydroxyle est substitué sur un carbone d'extrémité et l'autre sur l'atome de carbone à côté de celui-ci, et des mélanges de ceux-ci, et du butylcarbamate d'iodopropynyle.

2. Composition selon la revendication 1 dans laquelle les alcanediols en C₆₋₁₂ comprennent le 1,2-nonanediol et le 1,2-heptanediol.

3. Composition selon la revendication 1 dans laquelle le squelette 1,2-octanediol est en outre substitué par 1, 2 ou 3 groupes alkyle en C₁₋₄ et comprend le 3-méthyl-1,2-octanediol, le 4-méthyl-1,2-octanediol, le 3,4-diméthyloctanediol, le 3-éthyl-1,2-octanediol et le 4-éthyl-1,2-octanediol.

4. Composition selon l'une quelconque des revendications précédentes dans laquelle le rapport m/m du caprylyl glycol, de l'alcanediol en C₆₋₁₂ ou du 1,2-octanediol substitué par 1, 2 ou 3 groupes alkyle en C₁₋₄ au butylcarbamate d'iodopropynyle se situe dans la fourchette de 0,1 à 500.

5. Composition selon l'une quelconque des revendications précédentes dans laquelle le rapport m/m du caprylyl glycol, de l'alcanediol en C₆₋₁₂ ou du 1,2-octanediol substitué par 1, 2 ou 3 groupes alkyle en C₁₋₄ au butylcarbamate d'iodopropynyle se situe dans la fourchette de 20 à 75.

6. Composition selon l'une quelconque des revendications précédentes dans laquelle la concentration de caprylyl glycol, d'alcanediol en C₆₋₁₂ ou de 1,2-octanediol substitué par 1, 2 ou 3 groupes alkyle en C₁₋₄ se situe dans la fourchette de 0,2 à 10 % en poids.

7. Composition selon l'une quelconque des revendications précédentes dans laquelle la concentration de butylcarbamate d'iodopropynyle se situe dans la fourchette de 0,01 à 0,25 % en poids.

8. Composition selon l'une quelconque des revendications précédentes contenant environ 1 % en poids de caprylyl glycol et environ 0,018 % en poids de butylcarbamate d'iodopropynyle.

9. Composition selon l'une quelconque des revendications précédentes comprenant en outre des tensioactifs, des émulsifiants, des facteurs de consistance, des agents de conditionnement, des émollients, des ingrédients de soin de la peau, des hydratants, des épaississants, des lubrifiants, des charges, des antioxydants, des conservateurs, des fragrances, des anti-inflammatoires, des antibactériens ou des antifongiques.

10. Formulation topique comprenant une composition selon l'une des revendications précédentes.

11. Utilisation d'une composition selon les revendications 1 à 9 dans des formulations dermatologiques ou cosmétiques.
